# EUROPÄISCHE PATENTSCHRIFT

(11) **EP 1 122 231 B1**
(45) Veröffentlichungstag und Bekanntmachung des Hinweises auf die Patenterteilung: **23.04.2003**
(21) Anmeldenummer: 00127796.1
(22) Anmeldetag: 19.12.2000
(51) Int. Cl.: C07C 7/10

(54) **Verfahren zur Isolierung von Hochsiedern bei der Cyclooligomerisierung von 1,3-Butadien**
Process for the recovery of high boiling components from the cyclooligomerisation of butadiene-1,3
Procédé pour la récupération des produits à haut point d'ébullition lors de la cyclo-oligomérisation du butadiène-1,3

(30) Priorität: 03.02.2000 DE 10004758
(43) Veröffentlichungstag der Anmeldung: 08.08.2001
(73) Patentinhaber: Degussa AG, 40474 Düsseldorf (DE)
(72) Erfinder: Schiffer, Thomas, Dr., 45721 Haltern (DE); Wilczok, Norbert, 45481 Mülheim (DE); May, Matthias, 46282 Dorsten (DE); Oenbrink, Georg, Dr., 48249 Dülmen (DE)

(56) Entgegenhaltungen:
- GB-A- 1 287 252

## Beschreibung

Die Erfindung betrifft ein Verfahren zur Isolierung von hochsiedenden Monomeren aus dem Destillationsrückstand, insbesondere von C₁₆-Isomeren, die bei der katalytischen Cyclodimerisierung und Trimerisierung von 1,3-Butadien anfallen.

Die Umsetzung von 1,3-Butadien erfolgt technisch an Übergangsmetallkomplexen oder Übergangsmetallkomplexsalzen, die in der Regel homogen in einer unpolaren organischen Phase gelöst vorliegen. Als Lösemittel eignen sich inerte Kohlenwasserstoffe wie z. B. Benzol. Als Katalysator wird typischerweise ein Übergangsmetall der 4. bis 10. Gruppe des Periodensystems in der Oxydationsstufe 0 und gegebenenfalls ein Ligand wie z. B. ein Phosphin oder ein Phosphit verwendet (W. Brenner, P. Heimbach, H. Hey, E. W. Müller, G. Wilke, Liebigs Ann. Chem. 727, 1969, 161-182; DE 12 83 836, Studiengesellschaft Kohle; DE 19 42 729, Mitsubishi Petrochemical Company Ltd.). Je nach Katalysatorsystem bilden sich als Hauptprodukte entweder die Dimerisierungsprodukte Cyclooctadien (COD) und Vinylcyclohexen (VCH) oder das Trimerisierungsprodukt Cyclododecatrien (CDT). Die Selektivität der Umsetzung an den Katalysatoren ist hoch. Sie liegt in der Regel bei über 90 % zugunsten der jeweils gewünschten Hauptkomponente.

Nach der Umsetzung wird der homogene Katalysator zunächst zersetzt und abgetrennt. Typischerweise erfolgt die Zersetzung durch polare Lösemittel wie Monoalkohole mit 1 bis 6 Kohlenstoffatomen und Wasser oder durch verdünnte Säuren. Die Reaktionsmischung (organische Phase) wird anschließend destillativ aufgearbeitet. Dabei werden nicht umgesetztes 1,3-Butadien und Lösemittel in den Reaktionsprozess zurückgeführt; die verschiedenen Dimerisierungs- und Trimerisierungsprodukte werden anschließend destillativ voneinander getrennt.

Bei dem Kolonnensumpf handelt es sich um ein Produktgemisch, welches zum einen aus Oligomeren und Polymeren des 1,3-Butadiens besteht, zum anderen aber auch zahlreiche hochsiedende Monomere mit definierter Molekülstruktur aufweist.

Typische Monomere sind Verbindungen mit 12 bis 30 Kohlenstoffatomen, bestehend aus einem Ringgerüst von 6 bis 24 Kohlenstoffatomen und gegebenenfalls einer oder mehreren Seitenketten. Als wichtige, nicht limitierende Vertreter für solche Monomere seien 3-(2-Butenyl)-1,5,9-cyclododecatrien, 3-(3-Butenyl)-1,5,9-cyclododecatrien und 3-(1-Methylpropenyl)-1,5,9-cyclododecatrien sowie Cyclohexadeca-1,5,9,13-tetraen genannt.

Zahlreiche dieser makrozyklischen Monomeren konnten im Labormaßstab isoliert und ihre Struktur charakterisiert werden. Ausführliche Beschreibungen finden sich hierzu in DE 19 06 361 (Toyo Rayon Co.), GB 1 287 252 (Toray Industries), sowie in DE 20 63 348, US 3 658 926 und JP 48 019 304, zitiert nach CA: 79:78229 (alle Mitsubishi Petrochemical Co. Ltd.).

Schwierigkeiten bereitet in der Regel die selektive Synthese definierter Makrozyklen. Untersucht wurde z. B. die ringöffnende Metathese von 1,5-Cyclooctadien an Wolfram- (E.A. Ofstead, Macromol. Synth. 1977, 6, 69), Rhodium- (K. Saito et al., Bull. Chem. Soc. Jpn. 1979, 52, 3192) oder Rheniumkatalysatoren (US 3 865 888) oder die Oligomerisierung von 1,3-Butadien, bei der gegebenenfalls auch lineare Dimere wie 1,3,7-n-Octatrien zugegeben werden (DE 20 63 348). Bei allen Zugängen entsteht ein Produktgemisch, welches neben verschiedenen Makrozyklen auch Anteile an VCH, COD, CDT sowie Polybutadiene enthält.
Großtechnische Anwendung haben nur die selektiven Synthesen von CDT und COD gefunden.

Bei der selektiven Synthese von CDT bzw. COD bleibt im Sumpf der Destillationskolonne bei üblicher Form der Aufarbeitung ein hochsiedender, bisweilen gelblich gefärbter Rückstand zurück, der je nach Zusammensetzung bei Raumtemperatur entweder noch hochviskos zähflüssig bleibt oder wachsartig erstarrt vorliegt. Destillativ läßt sich aus diesem Rückstand mit vertretbarem Energieeintrag kein weiteres Wertprodukt mehr isolieren. Der Kolonnensumpf wird daher in der Regel als Abfallprodukt zur Verbrennung gegeben.

Einen Zugang zur Trennung der hochsiedenden Monomeren und polymeren Bestandteile bietet die Extraktion. In GB 1 287 252, Beispiel 1, erfolgt diese durch Zugabe großer Mengen Aceton (500 g) bezogen auf die Reaktionsmischung (162 g Butadien und 20 ml Toluol als Lösemittel). Auch in den anderen Beispielen von GB 1 287 252, in denen die Reaktionsmischung aufgearbeitet und nicht nur gaschromatographisch untersucht wird, wird die Extraktion mit Aceton gemäß Beispiel 1 angewandt.

In DE 19 06 361 werden als Lösemittel neben Toluol auch Hexan, Benzol, Ether (= Diethylether) und Tetrahydrofuran angegeben. Auch hierbei werden im Vergleich zum Lösemittel (in der Regel ca. 10 bis 50 ml) große Mengen Aceton (500 g) als Extraktionsmittel eingesetzt. Werden größere Volumina Lösemittel verwendet, 200 ml Toluol (Beispiel 10) oder 250 ml Hexan (Beispiel 15), so wird das Lösemittel destillativ reduziert, bevor Aceton als Extraktionsmittel zugesetzt wird. In allen Fällen verbleiben jedoch die Dimerisierungs- und Trimerisierungsprodukte COD, VCH und CDT in der Reaktionsmischung und werden folglich mit in Aceton aufgenommen. Auch JP 49 007 153 (zitiert nach CA: 81:13184) erwähnt Aceton zur Extraktion.

Die Anwendung der oben beschriebenen Extraktionsverfahren auf die selektive CDT- bzw. COD-Synthese bereitet jedoch erhebliche Schwierigkeiten. So fallen bei der direkten Zugabe von Aceton zur Reaktionsmischung die polymeren oder oligomeren Anteile nicht oder nur sehr unvollständig aus. Auch zeigte sich, dass die beschriebenen Lösemittel, insbesondere die aromatischen und etherischen Verbindungen, die oligomeren und polymeren Anteile gut lösen und damit die Extraktion stark erschweren.

Aufgrund der großen Mengen CDT bzw. COD, die bei der selektiven Synthese dieser Verbindungen mit zu extrahieren sind, wäre zudem die benötigte Menge Aceton bei einer Übertragung des Standes der Technik in einen technischen Maßstab enorm.

Es bestand daher die Aufgabe, ein Verfahren zur Isolierung der hochsiedenden Monomeren aus dem Destillationsrückstand, der bei der Cyclodimerisierung und/oder Cyclotrimerisierung von 1,3-Butadien nach Abtrennung der Zielprodukte Cyclooctadien, Vinylcyclohexen und/oder Cyclododecatrien anfällt, bereitzustellen, das die oben genannten Nachteile nicht aufweist.

Es wurde nun überraschend gefunden, dass sich die hochsiedenden Monomeren mit bestimmten Lösemitteln oder Lösemittelgemischen gut aus dem Destillationsrückstand extrahieren lassen, ohne dass dabei größere Mengen der oligomeren und polymeren Anteile gelöst werden. Die unlöslichen Oligomeren und Polymeren können dann vorwiegend in pulvriger, kristalliner und überraschend gut filtrierbarer Form abgetrennt werden. Vom Filtrat läßt sich das Extraktionsmittel bei passender Wahl vorzugsweise destillativ leicht von den extrahierten Monomeren abtrennen und die so gewonnenen Monomeren können dann vorzugsweise durch eine Vakuumdestillation in reiner Form gewonnen werden.

Gegenstand der Erfindung ist daher ein Verfahren zur Isolierung von hochsiedenden Monomeren aus dem Destillationsrückstand, der bei der Cyclodimerisierung und/oder Cyclotrimerisierung von 1,3-Butadien und Abtrennung der Zielprodukte anfällt, wobei
- die Leichtsieder (z. B. unumgesetztes 1,3-Butadien) und Lösemittel abgetrennt werden,
- die gewünschten Zielprodukte wie Cyclooctadien oder Cyclododecatrien destillativ gewonnen werden,
- der Destillationsrückstand mit einem unpolaren oder schwach polaren Lösemittel ausgewählt aus der Gruppe, die durch aliphatische, cycloaliphatische, olefinische oder cycloolefinische Kohlenwasserstoffe, Ketone oder Aldehyde oder deren Mischungen gebildet wird, extrahiert wird,
- unlösliche Oligomere und Polymere (zum Teil) kristallisieren und durch eine mechanische Trennoperation (z. B. Filtration) abgetrennt werden,
- das Extraktionsmittel vorzugsweise durch Destillation als Leichtsieder entfernt wird und
- die hochsiedenden Monomere vorzugsweise durch Vakuumdestillation in reiner Form gewonnen werden.

Bei der Untersuchung des Destillationsrückstandes durch Extraktion zeigte sich, dass diese sehr stark von der Wahl des Lösemittels abhängt. Überraschend wurde festgestellt, dass sich sowohl unpolare Lösemittel wie unverzweigte oder verzweigte aliphatische Kohlenwasserstoffe, vorzugsweise mit 5 bis 10 Kohlenstoffatomen (z. B. Pentan, Hexan und Octan), cycloaliphatische Kohlenwasserstoffe mit vorzugsweise 5 bis 10 Kohlenstoffatomen (z. B. Cyclohexan), unverzweigte oder verzweigte olefinische und cycloolefinische Kohlenwasserstoffe mit vorzugsweise 5 bis 12 Kohlenstoffatomen (z. B. Cycloocten) als auch einige schwach polare, aprotische Lösemittel wie gegebenenfalls verzweigte aliphatische und cycloaliphatische Ketone und Aldehyde mit vorzugsweise insgesamt 3 bis 12 Kohlenstoffatomen und deren Mischungen sehr gut als Extraktionsmittel eignen. Bevorzugte Lösemittel aus der Gruppe der Ketone sind Aceton und 2-Butanon (Methylethylketon). Auch überkritische Gase wie z. B. Kohlendioxid und Ethan sind prinzipiell als Extraktionsmittel geeignet.

Überraschend zeigte sich, dass die im Stand der Technik verwendeten Aromaten, Alkohole und Ether dagegen weniger gut geeignet sind. In der Regel ist in Alkoholen die Löslichkeit der Monomeren zu gering. In Aromaten und Ethern lösen sich die Oligomeren und Polymeren zu gut.

Das erfindungsgemäße Verfahren sieht nun vor, dass bei der Cyclodimerisierung bzw. Cyclotrimerisierung von 1,3-Butadien zunächst leicht flüchtige Komponenten abgetrennt werden und das jeweilige Hauptprodukt destillativ über Kopf von hochsiedenden Anteilen befreit wird. Dabei bleibt im Sumpf der Kolonne ein zäher Austrag an Hochsiedern zurück, der sich mit vertretbarem Aufwand nicht weiter destillieren läßt. Er wird nun erfindungsgemäß mit einem Lösemittel oder Lösemittelgemisch (Extraktionsmittel/-gemisch) digeriert, wobei vorzugsweise bei erhöhter Temperatur unter Rückfluss gearbeitet wird. Dabei lässt sich die Wärme des Austrags aus dem Sumpf der Kolonne zum Erwärmen des Extraktionsmittels ausnutzen. Nach dem Digerieren und Abkühlen werden die unlöslichen Komponenten durch eine mechanische Trerinoperation wie zum Beispiel eine Filtration oder Sedimentation abgetrennt. Das Lösemittel bzw. Lösemittelgemisch wird vom Extrakt vorzugsweise destillativ abgetrennt und kann erneut im Extraktionsprozess verwendet werden. In Sonderfällen kann auch eine chromatographische Abtrennung wie z. B. eine präparative Flüssigchromatographie (HPLC) erfolgen. Es bleibt ein hochsiedendes, oligomeren- und polymeren-armes Öl zurück, welches nun vorzugsweise im Vakuum weiter destillativ oder durch geeignete chromatographische Trennverfahren aufgereinigt werden kann.

Das Lösemittel oder Lösemittelgemisch wird für die Extraktion so gewählt, dass die monomeren Substanzen weitgehend aus dem Sumpf herausgelöst werden, während ein Großteil der oligomeren und polymeren Anteile unlöslich zurückbleibt. Durch die Wahl des richtigen Extraktionsmittels gelingt es ferner, dass der unlösliche Anteil des Sumpfes beim Digerieren zu kristallisieren beginnt. Durch gleichmäßiges Abkühlen der Extraktionsmischung lässt sich die Kristallisation erhöhen, wodurch der Anteil gelöster Polymere im Extrakt weiter verringert wird.

Der erhaltene Niederschlag besteht hauptsächlich aus Oligomeren und Polymeren des 1,3-Butadiens. Er ist in der Regel farblos und wenigstens zum Teil kristallin. Bei der Wahl des richtigen Extraktionsmittels und der richtigen Temperatur klebt er nicht nicht mehr oder nur wenig und lässt sich so leicht zum Beispiel über ein Filter oder eine Nutsche abtrennen.
Beim Abkühlen der Extraktionsmischung ergibt sich eine untere Temperaturgrenze dadurch, dass entweder die Löslichkeit der monomeren Anteile im Extraktionsmittel zu gering wird oder dass die Monomeren aus der Mischung ausfrieren. In beiden Fällen ist dies deutlich daran zu erkennen, dass die Extraktionsmischung milchig trüb wird und der Niederschlag verklebt. Die genaue Temperaturgrenze ist u. a. jeweils abhängig vom Extraktionsmittel und der Zusammensetzung der Hochsieder im Sumpf. Üblicherweise liegt sie zwischen + 20 °C und - 10 °C.

Der Siedebereich der zur Extraktion verwendeten Lösemittel soll zwischen 35 und 130 °C liegen. Als besonders bevorzugt hat sich ein Siedebereich von 50 bis 105 °C erwiesen. Dabei kann gegebenenfalls auch unter Druck gearbeitet werden.

Bei Lösemitteln mit Siedepunkten über 130 °C schmelzen die oligomeren und polymeren Anteile, was die Kristallisation dieser Komponenten bereits beim Digerieren unter Rückfluß verhindert. Bei Lösemitteln mit Siedepunkten unter 35 °C liegt der Siedepunkt des Extraktionsmittels zu nahe an der unteren Temperaturgrenze fiir die Löslichkeit der hochsiedenden Monomeren. Die gewünschten Eigenschaften des Extraktionsmittels lassen sich besonders gut auch durch Kombination zweier oder mehrerer Lösemittel einstellen.

Als ein weiterer Nebeneffekt des erfindungsgemäßen Verfahrens zeigt sich, dass restliche Anteile des Hauptproduktes, welche sich destillativ aus dem Sumpf der Kolonne nicht mehr isolieren lassen, durch Extraktion zugänglich gemacht werden. Es kann so weiteres Zielprodukt (Hauptprodukt) aus der Cyclodimerisierung oder Cyclotrimerisierung gewonnen werden.

Stammen die Hochsieder also beispielsweise aus dem Sumpf der Cyclododecatrien-Kolonne, so läßt sich durch die Extraktion weiteres Cyclododecatrien gewinnen.

Potentielle Anwendungsgebiete für diese hochsiedenden Monomeren liegen in der Parfüm- und Riechstoffindustrie, beispielsweise in der Synthese makrocyclischer Ketone (JP 57 021 254, Takasago Perfumery Co., zitiert in CA 97:162 457). Eine Hauptanwendung ergibt sich für diese Verbindungen jedoch als Vernetzer in synthetischen Kautschuken.

### Beispiele:

Das Verfahren soll durch die folgenden Beispiele näher erläutert werden, ohne jedoch auf die genannten Beispiele einzuschränken.

### Beispiele zur Extraktion:

Für die folgenden Beispiele wurde ein Destillationsrückstand aus der Cyclododecatrien-Herstellung verwendet. Dieser wurde jeweils in 400 ml Extraktionsmittel aufgenommen und 1 Stunde unter Rückfluß digeriert. Anschließend wurde die Mischung auf 20 °C bzw. 4 °C abgekühlt, über Nacht stehen gelassen und anschließend filtriert. Das Filtrat wurde vom Extraktionsmittel befreit und Filtrat und Filterkuchen ausgewogen. Die Verluste zwischen Einwaage und Auswaage ergaben sich durch Anhaftungen an den verwendeten Laborgeräten. Die Ergebnisse sind in der Tabelle 1 zusammengefaßt.

### Beispiel zur Destillation:

Ausgehend von Versuch 6 wurden 411,1 g Filtrat im Vakuum über eine 1 m Füllkörperkolonne destilliert. Es konnten noch 7,0 g CDT-Isomere (farbloses Öl, 78 bis 85 °C / 2 mbar) sowie 107,3 g einer Mischfraktion (hellgelbes Öl, 110 bis 122 °C / 2 mbar) und 238,2 g einer C₁₆-Hauptfraktion (farbloses Öl, 124 bis 126 °C / 2 mbar) isoliert werden. Der Rest blieb als dunkler Rückstand im Sumpf zurück. Die Analyse der C₁₆-Fraktion ergab 3-(2-Butenyl)-1,5,9-Cyclododecatrien als eines der Hauptprodukte.

**Tabelle 1:**

| **Nr.** | **Estraktions- mittel (Sdp in °C)** | **Einwaage Rückstand (g)** | **Filtration bei (°C)** | **Auswaage Filtrat (g)** | **Auswaage Niederschlag (g)** | **Aussehen Filtrat** | **Aussehen Niederschlag** |
|---|---|---|---|---|---|---|---|
| **1** | **n-Pentan (36)** | **151,3** | **4** | **120,4** | **22,1** | **gelbes Öl 1)** | **farblos, leicht klebrig** |
| **2** | **n-Hexan (68)** | **161,9** | **20** | **147,7** | **8,34** | **gelbes Öl, 1)** | **farblos, pulvrig** |
| **3** | **n-Heptan (98)** | **155,1** | **20** | **128,7** | **20,5** | **gelbes Öl** | **farblos, leicht klebrig** |
| **4** | **Petroleum- benzin (~100)** | **171,7** | **20** | **158,2** | **9,4** | **gelbes Öl 1)** | **farblos, pulvrig** |
| **5** | **Petroleum- benzin (~100)** | **148,2** | **4** | **83,7** | **54,2** | **gelbes Öl** | **farblos, klebrig** |
| **6** | **Aceton (56)** | **134,1** | **20** | **75,6** | **52,4** | **gelbes Öl** | **farblos, klebrig** |
| **7** | **2-Butanon (79)** | **166,4** | **20** | **144,9** | **11,8** | **gelbes Öl 1)** | **farblos, pulvrig** |
| **8** | **2-Butanon (79)** | **125,6** | **4** | **103,9** | **16,0** | **gelbes Öl** | **farblos, pulvrig** |
| **9** | **3-Pentanon (102)** | **150,9** | **4** | **96,3** | **40,1** | **gelbes Öl** | **farblos, pulvrig** |
| **10** | **Cyclopen- tanon (129)** | **154,3** | **4** | **122,3**^{**3)**} | **58,7** | **gelbes Öl** | **farblos, leicht klebrig** |
| **11** | **Propion- aldehyd (48)** | **150,8** | **4** | **53,0** | **97,2** | **gelbes Öl** | **gelb, pulvrig** |

| **Vergleichsbeispiele fiir weniger geeignete Lösemittel** | | | | | | | |
|---|---|---|---|---|---|---|---|
| **12** | **Isopropanol (82)** | **154,6** | **20** | **64,68** | **84,31** | **gelbes Öl** | **farblos, klebrig** |
| **13** | **Toluol (110)** | **163,2** | **20** | **--** | **--** | **gelbes Öl** | **- 2)** |
| **14** | **THF** | **148,3** | **20** | **--** | **--** | **gelbes Öl** | **- 2)** |

| | | | | | | | |
|---|---|---|---|---|---|---|---|
| 1) Filtrat klar, beim Abziehen des Extraktionsmittels bildet sich ein feiner, farbloser Niederschlag, | | | | | | | |
| 2) Vollständig gelöst, daher kein Niederschlag abtrennbar. | | | | | | | |
| 3) enthält noch ca. 10 % Extraktionsmittel | | | | | | | |

## Patentansprüche

1. Verfahren zur Isolierung von hochsiedenden Monomeren aus dem Destillationsrückstand, der bei der Cyclodimerisierung und/oder Cyclotrimerisierung von 1,3-Butadien nach Abtrennung der Zielprodukte Cyclooctadien, Vinylcyclohexen und/oder Cyclododecatrien anfällt,
**dadurch gekennzeichnet, dass**
- der Destillationsrückstand mit einem unpolaren oder schwach polaren Lösemittel, ausgewählt aus der Gruppe, die durch aliphatische, cycloaliphatische, olefinische oder cycloolefinische Kohlenwasserstoffe, Ketone oder Aldehyde oder deren Mischungen gebildet wird, extrahiert wird,
- unlösliche Oligomere und Polymere wenigstens zum Teil kristallisieren und durch eine mechanische Trennoperation abgetrennt werden
- das Extraktionsmittel entfernt wird und
- die hochsiedenden Monomeren gewonnen werden.

2. Verfahren nach Anspruch 1
**dadurch gekennzeichnet,**
**dass** das Lösemittel oder Lösemittelgemisch für die Extraktion einen Siedepunkt zwischen 35 °C und 130 °C hat.

3. Verfahren nach Anspruch 1
**dadurch gekennzeichnet,**
**dass** das Lösemittel oder Lösemittelgemisch für die Extraktion einen Siedepunkt zwischen 50 °C und 105 °C hat.

4. Verfahren nach Anspruch 1,
**dadurch gekennzeichnet,**
**dass** als Lösemittel für die Extraktion Aceton eingesetzt wird.

5. Verfahren nach Anspruch 1,
**dadurch gekennzeichnet,**
**dass** als Lösemittel für die Extraktion 2-Butanon eingesetzt wird.

6. Verfahren nach einem der Ansprüche 1 bis 5,
**dadurch gekennzeichnet,**
**dass** die Abtrennung der unlöslichen Komponenten nach dem Digerieren durch Sedimentation erfolgt.

7. Verfahren nach einem der Ansprüche 1 bis 5,
**dadurch gekennzeichnet,**
**dass** die Abtrennung der unlöslichen Komponenten nach dem Digerieren durch Filtration erfolgt.

8. Verfahren nach einem der Ansprüche 1 bis 7,
**dadurch gekennzeichnet,**
**dass** aus dem Extrakt weiteres Zielprodukt aus der Cyclodimerisierung oder Cyclotrimerisierung gewonnen wird.

9. Verfahren nach einem der Ansprüche 1 bis 7,
**dadurch gekennzeichnet,**
**dass** aus dem Extrakt eine Hochsiederfraktion gewonnen wird, die 3-(2-Butenyl)-1,5,9-cyclodo-decatrien, 3-(3-Butenyl)-1,5,9-cyclododecatrien, 3-(1-Methylpropenyl)-1,5,9-cyclododeca-trien und / oder Cyclohexadeca-1,5,9,13-tetraen enthält.

## Claims

1. A process for the isolation of high-boiling monomers from the distillation residue formed in the cyclodimerization and/or cyclotrimerization of 1,3-butadiene after the target products cyclooctadiene, vinylcyclohexene and/or cyclododecatriene have been separated off, **characterized in that**
- the distillation residue is extracted with a nonpolar or slightly polar solvent selected from the group formed by aliphatic, cycloaliphatic, olefinic or cycloolefinic hydrocarbons, ketones or aldehydes or mixtures thereof,
- insoluble oligomers and polymers crystallize at least partly and are separated off by a mechanical separation operation,
- the extractant is removed and
- the high-boiling monomers are isolated.

2. A process according to claim 1, **characterized in that** the solvent or solvent mixture for the extraction has a boiling point in the range from 35°C to 130°C.

3. A process according to claim 1, **characterized in that** the solvent or solvent mixture for the extraction has a boiling point in the range from 50°C to 105°C.

4. A process according to claim 1, **characterized in that** the solvent used for the extraction is acetone.

5. A process according to claim 1, **characterized in that** the solvent used for the extraction is 2-butanone.

6. A process according to any one of claims 1 to 5, **characterized in that** the insoluble components after the digestion are separated off by sedimentation.

7. A process according to any one of claims 1 to 5, **characterized in that** the insoluble components after the digestion are separated off by filtration.

8. A process according to any one of claims 1 to 7, **characterized in that** further target product of the cyclodimerization or cyclotrimerization is isolated from the extract.

9. A process according to any one of claims 1 to 7, **characterized in that** a high boiler fraction comprising 3-(2-butenyl)-1,5,9-cyclododecatriene, 3-(3-butenyl)-1,5,9-cyclododecatriene, 3-(1-methylpropenyl)-1,5,9-cyclododecatriene and/or cyclohexadeca-1,5,9,13-tetraene is isolated from the extract.

## Revendications

1. Procédé d'isolement de monomères à point d'ébullition élevé provenant de résidu de distillation qui se forment dans la cyclodimérisation et/ou la cyclotrimérisation du 1,3-butadiène après séparation des produits cibles, le cyclooctadiène, le vinylcyclohexène et/ou le cyclododécatriène,
**caractérisé en ce que**
- le résidu de distillation est extrait avec un solvant non polaire ou faiblement polaire choisi dans le groupe qui est formé d'hydrocarbures aliphatiques, cycloaliphatiques, oléfiniques ou cyclooléfiniques, des cétones ou des aldéhydes ou de leurs mélanges,
- des oligomères et des polymères insolubles cristallisent au moins en partie et sont séparés par une opération de séparation mécanique,
- l'agent d'extraction est éliminé et
- les monomères à point d'ébullition élevé sont récupérés.

2. Procédé selon la revendication 1,
**caractérisé en ce que**
le solvant ou le mélange de solvants pour l'extraction a un point d'ébullition compris entre 35°C et 130°C.

3. Procédé selon la revendication 1,
**caractérisé en ce que**
le solvant ou le mélange de solvants pour l'extraction a un point d'ébullition compris entre 50°C et 105°C.

4. Procédé selon la revendication 1,
**caractérisé en ce que**
comme solvant pour l'extraction on met en oeuvre de l'acétone.

5. Procédé selon la revendication 1,
**caractérisé en ce que**
comme solvant pour l'extraction on met en oeuvre de la 2-butanone.

6. Procédé selon l'une des revendications 1 à 5,
**caractérisé en ce que**
la séparation des composants insolubles après la digestion, s'effectue par sédimentation.

7. Procédé selon l'une des revendications 1 à 6,
**caractérisé en ce que**
la séparation des composés insolubles après la digestion s'effectue par filtration.

8. Procédé selon l'une des revendications 1 à 7,
**caractérisé en ce qu'**
à partir de l'extrait, davantage de produit cible provenant de la cyclodimérisation ou de la cyclotrimérisation est obtenu.

9. Procédé selon l'une des revendications 1 à 7,
**caractérisé en ce qu'**
à partir de l'extrait on obtient une fraction de produit à haut point d'ébullition qui renferme du 3-(2-butényl)-1,5,9-cyclododécatriène, du 3-(3-butényl)-1,5,9cyclododécatriène, du 3-(1-méthylpropényl)-1,5,9-cyclododécatriène et/ou du cyclohexadéca-1,5,9,13-tétraène.
